# EUROPEAN PATENT APPLICATION

(11) **EP 4 108 325 A1**
(43) Date of publication of application: **28.12.2022**
(21) Application number: 21181245.8
(22) Date of filing: 23.06.2021
(51) Int. Cl.: B01J 3/00, B01J 19/08, B01J 19/12, A01C 1/08, H05H 1/32, B01J 3/03

(54) **METHOD AND DEVICE FOR TREATMENT OF AGRICULTURAL PRODUCTS WITH COLD PLASMA**

(71) Applicant: Jozef Stefan Institute, 1000 Ljubljana (SI); Interkorn d.o.o., 9231 Beltinci (SI)
(72) Inventor: GSELMAN, Peter, 9231 Beltinci (SI); MOZETIC, Miran, 1000 Ljubljana (SI); PRIMC, Gregor, 1000 Ljubljana (SI); RECEK, Nina, 1000 Ljubljana (SI)
(74) Representative: Mewburn Ellis LLP

(57) **Abstract**

The present invention relates to a method and device for the rapid degradation of toxins likely to be synthesized by moulds upon improper storage of different agricultural products like grains, seeds, kernel, nuts, or beans. The products are detoxified by brief exposure to a gaseous plasma created in a hydrogencontaining atmosphere at low pressure.

## Description

### Field of the Invention

The present invention relates to a method and device for treatment of agricultural products and particularly, although not exclusively, to a method and device for destruction of mould-born toxins on the surface of agricultural products, in particular seeds, with gaseous plasma at low pressure. The seeds contaminated with toxins are, for example, dropped through inductively coupled gaseous plasma sustained in a hydrogen-containing gas. The treatment, according to the methods of the invention, is useful for detoxification of various seeds, nuts, beans, kernel, and similar agricultural products. The treated products are suitable for food or animal feedstock.

### Background

Improper storage of seeds, nuts, beans, and similar agricultural products leads to a proliferation of different microorganisms, in particular moulds. Some varieties of microorganisms produce toxins, i.e., organic molecules that suppress the proliferation of other microorganisms. In particular, some varieties of mould produce toxins that are not only harmful to other microorganisms but also humans and animals. The agricultural products contaminated with a significant amount of toxins are not useful for food or feedstock since the toxins cause serious health issues. Some toxins are even carcinogenic.

The toxins produced by moulds upon improper storage (in particular high humidity and elevated temperature) of the agricultural products cannot be disintegrated or inactivated by cooking since they are thermally very stable even at autoclaving conditions, i.e., temperature around 130 °C and elevated pressure. Other techniques have been proposed for chemical modification using aggressive reagents such as ammonia or ozone. The chemical techniques enable at least partial destruction of toxins, but the required treatment times are prohibitively long. The chemical destruction often takes hours, if not days, so they have not been used in large facilities.

Agricultural products have been treated by gaseous plasma to decrease the concentration of toxins and disinfect the products. For example, JP2019209170 (A) describes a method where an atmospheric pressure plasma sustained with a dielectric barrier discharge is used. The device comprises a grounded electrode, an ungrounded electrode, and a dielectric is installed inside a reaction vessel provided with an inlet and an outlet for a plasma processing object. The centre of the reaction vessel is rotated by a driving system for rotating it about the centre axis thereof as a rotation axis. Plasma processing is performed while the processing object is rotated and stirred. A grounded electrode having a rectangular or triangular shape in cross-section and an ungrounded electrode made of a metal wire covered with a dielectric are used in JP2019209170 (A). The rotation or stirring is essential to assure for rather homogeneous treatment of large quantities of agricultural products.

US2020071199 (A1) describes an apparatus, method, and system used to treat various liquids, seeds, and soil or other growth media used in cultivating plants by exposure to a plasma discharge. The system comprises a novel electrode structure that utilizes a tapered inner electrode and a porous outer electrode to create a three-dimensional plasma discharge, which is applied to the liquid, seeds, or growth media through removable and attachable sub-assemblies. When used to treat water or seeds, highly-concentrated plasma-activated water (PAW) and disease and drought-resistant plasma-activated seeds (PAS) are produced, which improve the germination rate, efficiency, and eliminate the need for ammonia-based chemical fertilizers. The plasma arc is sustained at atmospheric (or somehow higher) pressure.

SK792018 (A3) employs a low-temperature plasma in combination with a subsequent surface treatment of the seed using a mordant, wherein the low-temperature plasma is generated at atmospheric pressure in air or other conventional working gas. The mordant can be applied at a lower dose after the plasma treatment of the seeds. Further described is an apparatus enabling the method to be carried out, comprising a block for the surface treatment of seeds by plasma and a block for the subsequent treatment of seeds with seed dressing.

RU2705791 (C1) describes low-temperature non-equilibrium argon plasma at atmospheric pressure, which was found useful for sterilization/disinfection of medical instruments and accessories, microorganisms (bacteria, spores, pathogenic microflora) disinfection, in particular, during storage, drying or pre-sowing. In particular, the treatment of agricultural products (seeds, vegetables, fruits, fodder mixtures) is disclosed. The source of non-equilibrium argon plasma based on volumetric glow discharge of atmospheric pressure comprises an electrode system of pin cathodes and a flat anode with the gas flow in the discharge gap, a high-voltage power supply, and a gas injection system.

JP2019162070 (A) provides sterilization equipment that can sequentially sterilize a large number of seeds in an open space when sterilizing the seeds by using plasma at atmospheric pressure. Sterilization equipment includes a gas plasma generator including at least one pair of electrodes in a gas flow path where the gas serving as a plasma source flows through, generating gas plasma by applying AC voltage between the pair of electrodes, and discharging the generated gas plasma from an exit out of gas flow path to the air to form a continuation movement area of the gas plasma toward the exit outward, and holding seeds in the continuation movement area of this gas plasma generator. The gas plasma is applied from different directions to the seeds held by the holding means, and thereby sterilization is performed.

CN109511311 (A) describes a low-temperature plasma seed treatment machine for agriculture and animal husbandry. It comprises a box body, a base table, and an insulator medium tube. A feeding device is arranged on one side of the top of the box body and comprises a hopper, a cylindrical shell, and a conveying roller. An insulator medium tube is mounted on a mobile device, which comprises a forward and reverse rotating motor, a threaded rod, and a movable block; a supporting rod is fixed to the position at the bottom of an inner cavity of the box body.

CN109463062 (A) provides a method for treating crop seeds by atmospheric pressure room temperature plasma. The method can be used for treating mung bean seeds, soybean seeds, corn seeds, and wheat seeds. Compared with the control group, the bud ratio of crop seeds treated by plasma is obviously improved. The bud ratio of crop seeds subjected to germination treatment is further improved, and the mutagenic effect is better as well. For example, the bud ratio of crop seeds subjected to mechanical seed-coat breaking treatment is higher than that of crop seeds subjected to seed coat complete germination, and the bud ratio of crop seeds subjected to gibberellin soaking treatment is higher than that of un-soaked crop seeds.

CN109121550 (A) relates to an oryza sativa breeding method by microwave plasma treatment. The oryza sativa breeding method by microwave plasma treatment has potential advantages such as possible prolonged flowering time of the sterile lines, a possible improvement on yield, resistance and quality, shortened breeding period, and the new oryza sativa species excellent in comprehensive characters can be bred.

There are several scientific papers on plasma treatment of seeds. All authors employed atmospheric-pressure plasma treatment and report significant improvements in terms of toxin degradation. Treatment times of several minutes have been found particularly useful to decrease the concentration of toxins below the level tolerable for using the agricultural products for food or feedstock. The papers include:
Basaran, P., Basaran-Akgul, N., & Oksuz, L. (2008). Elimination of Aspergillus parasiticus from nut surface with low-pressure cold plasma (LPCP) treatment. Food Microbiology, 25(4), 626-632.
Park, B. J., Takatori, K., Sugita-Konishi, Y., Kim, I.-H., Lee, M.-H., Han, D.-W., ... Park, J.-C. (2007). Degradation of mycotoxins using microwave-induced argon plasma at atmospheric pressure. Surface and Coatings Technology, 201(9-11), 5733-5737.
Shi, H., Cooper, B., Stroshine, R. L., Ileleji, K. E., & Keener, K. M. (2017). Structures of degradation products and degradation pathways of aflatoxin B1 by high-voltage atmospheric cold plasma (HVACP) treatment. Journal of Agricultural and Food Chemistry, 65(30), 6222-6230.
Shi, H., Ileleji, K., Stroshine, R. L., Keener, K., & Jensen, J. L. (2017). Reduction of aflatoxin in corn by high voltage atmospheric cold plasma. Food and Bioprocess Technology, 10(6), 1042-1052.
Siciliano, I., Spadaro, D., Prelle, A., Vallauri, D., Cavallero, M., Garibaldi, A., & Gullino, M. (2016). Use of cold atmospheric plasma to detoxify hazelnuts from aflatoxins. Toxins, 8(5), 125.
Kutasi, K., Popovic, D., Krstulovic, N., & Milošević, S. (2019). Tuning the composition of plasma-activated water by a surface-wave microwave discharge and a kHz plasma jet. Plasma Sources Science and Technology, 28(9), 095010.
Laroussi, M. (2002). Nonthermal decontamination of biological media by atmospheric pressure plasmas: review, analysis, and prospects. IEEE Transactions on Plasma Science, 30(4), 1409-1415.

Plasma at low pressure is rarely used. CN110622648 (A) describes a seed treatment method comprising the steps: putting seeds into a vacuum device, establishing vacuum in the device, and then filling it with helium, allowing a cold plasma generator arranged inside the vacuum device to operate and generate vacuum ultraviolet rays to irradiate the seeds so that biological macromolecules of the seeds experience energy transition. It also provides the cold plasma test platform, which comprises a vacuum device, a cold plasma generator and a sample tray arranged in the vacuum device. The vacuum device is externally connected with a vacuum pump and a helium gas source. The cold plasma generator comprises a polar plate arranged inside the vacuum device and a radio frequency generator externally connected to the polar plate. The gaseous plasma is sustained in helium using an RF discharge in the capacitive mode.

CN110192456 (A) provides methods for continuous-treatment seed sterilization. The cold plasma is used for the treatment of fruit and vegetable surfaces as well as seed. Processed corn seeds are sterilized through the vacuum cold plasma technology. The corn seeds are put on a conveying device to penetrate through an atmospheric pressure cold plasma sterilizing bin, and non-ionizing radiation treatment is conducted on the corn seeds for 15-20 seconds under the treatment power of 0-280 W. Streamline operation can be conducted, and the treatment efficiency was reported high. Also, it was shown through research that the method could specifically improve the saline-alkaline-tolerant growth performance of the corn seeds. The healthy seeds are obtained through a physical method, the damage of the soil to the seed coating is reduced, the consumption of pesticides is reduced, and the method has high promoting significance.

KR101976164 (B1) provides a plasma seed treatment apparatus, which comprises: a magnetron antenna in which microwaves are generated, a plurality of vacuum chambers in which the microwaves propagate, a plasma induction antenna disposed at the centre of the vacuum chambers to generate plasma, and a stirring chamber positioned inside the vacuum chamber that rotates to stir seeds. Power levels up to 80 W are disclosed, and the useful treatment times are in the order of several minutes.

A disadvantage of the techniques described in state of the art is the long treatment time needed. Typically, the treatment time that assures a reasonable decrease in the toxin concentration is in the order of several minutes, which is too long and not practical for mass application.

There remains a need to develop a method which enables a more rapid destruction of toxins on agricultural products.

The present invention has been devised in the light of the above considerations.

### Summary of the Invention

The present inventors have produced a method and device for the rapid removal of toxins and contaminants on various agricultural products using gaseous plasma sustained in a hydrogen-containing gas at low pressure. This means that the treatment time may be short enough for practical mass application, while also not compromising the effectiveness of the plasma treatment.

As is already known, plasma may be used to destroy toxins and contaminants. The characteristics of the plasma is dependent on several parameters which ultimately have an impact on the efficacy of toxin destruction. The inventors have devised a way to produce a plasma under certain conditions to improve upon the long treatment time described in the art. Specifically, the plasma is sustained in a gas mixture containing hydrogen, which is found to be sufficiently radiation intensive for the treatment. The plasma is sustained within a discharge power density range of 500 to 10000 W/litre. This range allows for a sufficient density of charged and neutral particles and ultraviolet radiation in the plasma for the treatment, while preventing any extensive thermal damage to the products themselves. The plasma is also sustained at a low gas pressure of 1 to 10000 Pa. Hydrogen plasma at such a low pressure radiates in the far-UV radiation range which is most effective for breaking the bonds of organic matter of the toxins because the electron temperature is higher than that of atmospheric pressure hydrogen plasma. It is these conditions that allow the treatment time to be significantly reduced, to the order of seconds.

Therefore, discussed herein is a method for detoxifying agricultural products contaminated with toxins with gaseous plasma at low pressure. The agricultural products, which may be seeds, kernel, grains, nuts and beans, contaminated with toxins may be dropped through the plasma sustained in hydrogen, which provides an effective treatment because of the short time needed by the present methods. Such a dropping method is advantageous for ease of practical application. Also discussed herein is a device for detoxifying agricultural products contaminated with toxins, which is enabled by the present method.

According to the first aspect of the invention, there is provided a method for detoxifying agricultural products contaminated with toxins, the method comprising subjecting the products to gaseous plasma sustained in an atmosphere comprising hydrogen, the plasma being sustained at a discharge power density of 500 to 10000 W/litre at a gas pressure of 1 to 10000 Pa.

In some embodiments, the plasma is sustained at a discharge power density of 1000 to 4000 W/litre.

In some embodiments, the plasma is sustained at a gas pressure of 10 to 1000 Pa, more preferably 30 to 100 Pa.

In some embodiments, the plasma is sustained with an inductively coupled radiofrequency discharge, such that plasma discharge occurs in the H-mode, or with a microwave discharge.

In some embodiments, the atmosphere for sustaining the plasma comprises at least 10 % hydrogen, preferably at least 50 % hydrogen, more preferably at least 80 % hydrogen, most preferably is substantially pure hydrogen.

In some embodiments, the atmosphere is derived from a working gas which comprises a hydrogen-containing gas selected from hydrogen water vapour, hydrogen sulphide, and ammonia, or a mixture of two or more of these.

In some embodiments, the time for which the products are subjected to the plasma is 0.1 to 100 s, preferably 0.3 to 3 s.

In some embodiments, the method comprises dropping the products from a first position to a second position through a treatment zone, wherein while the products are falling through the treatment zone they are subjected to the plasma.

In some embodiments, the agricultural products are seeds, kernel, grains, nuts and beans in any form, original, ground, or milled.

According to the second aspect of the invention, there is provided a device for detoxifying agricultural products contaminated with toxins, the device comprising: (a) a vacuum-tight discharge chamber which is controllably connected to a source of reactive hydrogen species and (b) an inductively coupled radiofrequency generator or a microwave generator, wherein the inductively coupled radiofrequency generator, where present, is connected to a coil via a matching network to sustain plasma within the discharge in use, the coil mounting on the discharge chamber, or wherein the microwave generator, where present, houses the discharge chamber.

In some embodiments, the discharge chamber is made from a dielectric material.

In some embodiments, the discharge chamber comprises an inlet and an outlet for passing through agricultural products, wherein the coil, where present, is positioned between the inlet and the outlet, such that, in use, a product can fall through the discharge chamber from the inlet to the outlet passing the location of the coil.

In some embodiments, the discharge chamber has a cylindrical shape.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1** shows a schematic of a device useful for employing the methods of the invention.
**Figure 2** shows a schematic of the process of detoxification of a seed with gaseous plasma.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

Gaseous plasma is a state of gas that consists of gaseous molecules, radicals, and charged particles, in particular positively charged ions and free electrons. It may also be a rich source of ultraviolet radiation. Gaseous plasma can be sustained at various pressures in various gases. Particularly popular are atmospheric-pressure plasmas and plasmas sustained at low-pressure conditions, especially when the product of gas pressure and distance between the powered and the grounded electrode is close to the Paschen minimum of breakdown voltage, about a few cm mbar.

Gaseous plasma at atmospheric pressure could be close to or far from thermal equilibrium. Gaseous plasma close to thermal equilibrium is hot plasma and gaseous plasma far from thermal equilibrium is cold plasma. Cold plasma is sustained at atmospheric pressure only by high-impedance discharges, such as a corona discharge or dielectric barrier discharge (DBD). When the impedance of the discharge is lowered (i.e., the electrical conductivity of gaseous plasma increases), the plasma becomes hotter until it suddenly transforms into a low-impedance discharge and becomes hot plasma. The transition from high-to low-impedance discharge is possible only when the electrical circuit and, in particular, the properties of the power supply support it. The hot plasma sustained by low-impedance atmospheric pressure discharge is often called an electric arc and previously has not been found useful for detoxification of agricultural products. One reason is that the gas temperature in electric arcs is often about 10000 K, so the products may take extensive thermal damage (they might even burn). The volume of such an electric arc is small, typically a few cm³. An alternative to the electric arc is a plasma torch where hot plasma is often sustained by electrodeless discharges, such as radiofrequency or microwave discharges. The hot plasma in atmospheric pressure torches is also limited to a small volume and, as such, hot plasma is found to be unsuitable for detoxification of agricultural products.

Electron streamers sustain the cold plasma at atmospheric pressure. The streamers are of short duration, typically a microsecond, and cause the formation of plasma in a short volume for a short time. Numerous streamers are often observed in cold atmospheric pressure high-impedance discharges, so the gas between the electrodes resembles non-equilibrium gaseous plasma. Cold atmospheric pressure plasmas previously have not been found useful for detoxification of agricultural products since it was not possible to sustain a temporally-averaged high density of reactive plasma species in a large volume useful for the treatment of large quantities of agricultural products. Cold atmospheric pressure plasma was found useful for academic research.

Among low-pressure plasmas, various discharge configurations were found useful for various applications.

There is currently no method of agricultural product treatment in the art which involves the use of gaseous plasma sustained in a hydrogen-containing gas mixture at low pressure and under conditions which achieve rapid and effective detoxification within the order of seconds. The present inventors have found that such a plasma can be used successfully under certain conditions.

Effective detoxification should ideally be performed in a shorter treatment time to avoid heating the seeds' interior. On the other hand, the treatment should also be performed in a sufficiently powerful discharge to assure for extensive surface reactions in short treatment time. Such treatment cannot be achieved just by any gaseous plasma. For plasma to be suitable for the application of detoxification, it is assumed that the plasma should be rich in chemically reactive species such as atoms and ultraviolet radiation, and cold enough to prevent significant heating of the interior of agricultural products.

The present inventors have investigated hydrogen as a useful plasma species. According to a first aspect of the present invention, there is provided a method for detoxifying agricultural products contaminated with toxins, the method comprising subjecting the products to gaseous plasma sustained in an atmosphere comprising hydrogen, the plasma being sustained at a discharge power density of 500 to 10000 W/litre at a gas pressure of 1 to 10000 Pa.

A useful discharge for sustaining gaseous plasma of such properties in continuous mode is the inductively coupled radiofrequency discharge in the H-mode (ICP). The power density of such a discharge is high enough to assure for almost full dissociation of gaseous molecules to radicals and, on the other hand, low enough to prevent the transformation of the discharge to an electric arc of extremely high gas temperature.

Therefore the plasma in the present invention is preferably sustained with inductively coupled radiofrequency discharge in the high-confinement mode or H-mode (thereafter: ICP). Such plasma may alternatively be sustained with a microwave discharge using a microwave generator.

According to the methods of the invention, plasma of appropriate parameters may be sustained at a discharge power density of 500 to 10000 W/litre, more preferably at a discharge power density of 1000 to 4000 W/litre. The discharge power density range assures that the discharge power density is not too low to allow sufficient dissociation of gas molecules and to allow for plasma to be sufficiently sustained, and not too high to prevent the formation of hot plasma which would otherwise significantly damage the products themselves.

As mentioned above, the discharge power density range may be achieved using either a radiofrequency generator or a microwave generator, preferably a radiofrequency generator. Various different frequencies may be useful. For example, a frequency of 13.56 MHz and even 27.12 MHz were found to be almost as useful as the frequency of 150 kHz. A powerful non-equilibrium gaseous plasma can also be sustained in the desired range of absorbed power density using microwave sources. Plasma sustained with a microwave source is capable of absorbing as much discharge power as a radiofrequency plasma.

Under the preferred conditions of the discharge power density, a moderate ionization fraction and a large dissociation fraction may be achieved, wherein the density of charged particles in ICP may be between 10¹⁸ and 10¹⁹ m⁻³. The density of neutral reactive particles such as H-atoms may preferably be more than 10²⁰ m⁻³, more preferably more than 10²¹ m⁻³. The density of charged particles is often measured with an electrical probe, in particularly with a double-electrode probe kept close to the floating potential. The density of H-atoms is often measured by optical absorption techniques or a catalytic probe. It should be noted that, according to the methods of the invention, the density range may be achieved as a direct consequence of the preferred discharge power density and pressure applied.

Hydrogen is the preferred working gas in the gas mixture used to generate the plasma. It is found that plasma sustained in pure hydrogen is more radiation intensive than plasma sustained in water-vapour. The degradation rate of toxins is greater for hydrogen plasma than for water-vapour plasma. The inventors believe that the destruction of the toxins is due to the synergy between ultraviolet radiation of the plasma and the chemical interaction between hydrogen atoms and the toxins. The UV radiation breaks bonds in the surface film of the organic material (i.e. toxins), and the dangling bonds are attacked by H-atoms, thus preventing recovery of the original chemical bonds in the organic matter of the toxin. In preferred embodiments, at least 10 % hydrogen is present in the gas mixture used to generate the plasma (sometimes called working gas) for this synergy. The working gas may also comprise water-vapour, hydrogen sulfide or ammonia, or a mixture of two or more of these gases. The working gas may preferably comprise at least 20 % hydrogen, more preferably at least 50% hydrogen, and yet more preferably at least 80 % hydrogen. The most preferred embodiment is use of substantially pure hydrogen as the working gas. It will be appreciated that, in some practical cases, water vapour may be present in the vacuum system, so a pure hydrogen atmosphere may not always be achieved. According to the methods of the invention, the plasma electrons will excite hydrogen molecules and atoms to highly excited states, which will relax by radiation in the far-ultraviolet range. Such radiation will break bonds in the surface film of organic materials.

A significant amount of gaseous species other than hydrogen in the gas mixture may contribute to the loss of electron energy. For example, when water vapour is added, the major source of loss of electron energy will be the dissociation of the H₂O molecules to H and OH and excitation of the resultant OH radicals to resonant state. The resonant state will almost immediately relax by radiation in the UV-B range, where the photon energy is not sufficient enough for extensive breaking bonds in the organic matter. A significant part of absorbed discharge power will be therefore spent in reactions that do not lead to excitation of hydrogen molecules or atoms. A similar result may also apply for ammonia or hydrogen sulfide, or other hydrogen-containing gases.

Accordingly, use of a working gas which is water vapour, hydrogen sulfide or ammonia, or any other hydrogen-containing gas, alone will lead to a plasma sustained in an atmosphere comprising hydrogen: hydrogen will have been formed by dissociation of the working gas.

That is, for example, where the working gas is water vapour dissociation leads to an atmosphere in which H, OH, H₂ and O are present. Accordingly the plasma is ultimately sustained in an atmosphere that comprises hydrogen. Most preferably, the plasma is sustained in a pure hydrogen atmosphere.

Low gas pressure is used to sustain the plasma. Although ICP can be sustained in a broad range of pressures, the gas pressure is preferably between 1 and 10000 Pa, more preferably between 10 and 1000 Pa, and most preferably between 30 and 100 Pa. The skilled person should appreciate that a low gas pressure in this context may refer to any value of pressure below standard atmospheric pressure. Low pressure hydrogen plasma radiates in the far-UV radiation range which is most effective for breaking the bonds of organic matter of the toxins because the electron temperature is higher than that of atmospheric pressure hydrogen plasma. Furthermore, far-UV radiation is less absorbed by gaseous atoms and molecules at low pressure than at atmospheric pressure because the penetration depth of far-UV radiation in the gas phase is inversely proportional to the pressure. The penetration depth of far-UV radiation in the gas phase may be about 1 mm at 1bar and a few cm at 10 mbar. Therefore, cold plasma at atmospheric pressure may not be preferred for use in the application of detoxifying agricultural products.

In further preferred embodiments, the gas temperature within the discharge is between about 300 and 3000 K, most preferably around 1000 K. Such a temperature is low enough to prevent degradation of agricultural products, for example upon falling through the plasma in a discharge chamber. Such a temperature is also within the definition and operation of cold plasma, wherein the gas temperature is typically lower than around 10000 K. It should be noted that, according to the methods of the invention, such a temperature may be achieved as a direct consequence of the preferred discharge power density and pressure applied.

The preferred treatment time of the agricultural products is in the order of seconds, preferably between 0.1 s and 100 s, more preferably between 0.3 s and 3 s. Such short times are enabled by the present invention. According to the method of the present invention, the short treatment time is advantageous in that it allows for rapid destruction of toxins and contaminants which is necessary for mass application of the method. The short treatment time may be used with the preferred conditions to assure that the toxin concentration on the agricultural products can be reduced significantly. Furthermore, the short treatment time couples with the above mentioned preferred gas temperatures to further suppress degradation of the agricultural products.

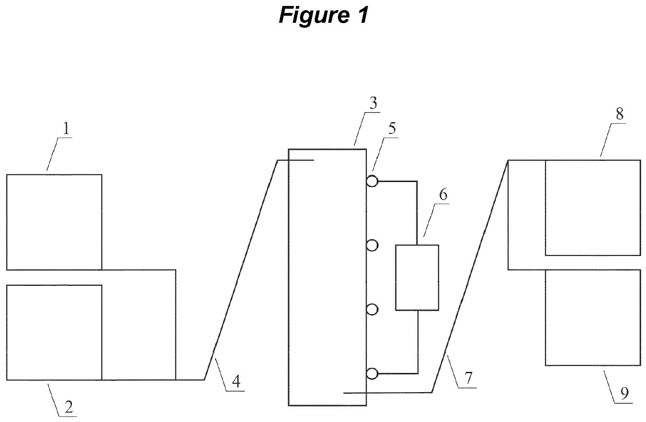

The present methods may optionally comprise some step by which the agricultural products are moved through a treatment zone in which they are exposed to the aforementioned plasma. This can allow an almost continuous treatment process instead of a batch process, yet further increasing the practicality of the process. For example, the method might involve dropping the agricultural products from a first position to a second position through a treatment zone, wherein while the products are falling through the treatment zone they are subjected to the plasma. The time taken for a product to fall through the treatment zone may be equal to the treatment time if the products fall exactly once. The products may be dropped through the treatment zone multiple times for sufficient destruction of toxins if necessary. A suitable device for this type of method is illustrated in Figure 1, discussed below.

The agricultural products are not particularly limited, but may suitably be seeds, kernel, grains, nuts or beans in any form, original, ground, or milled.

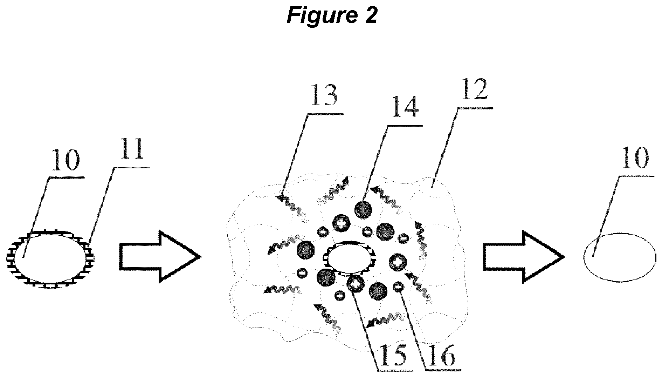

For the purpose of illustration of the general concept, a schematic showing the process of detoxification of a seed with gaseous plasma is shown in Figure 2. A seed 10 contaminated with toxins 11 is exposed to gaseous plasma 12, consisting of radiation 13, neutral atoms and excited atoms and molecules 14, ions 15, and electrons 16. These interact with the toxins 11, removing and destroying them to leave the seed 10 or other agricultural product without the toxins.

The scope of toxins and contaminants which may be removed from agricultural products is not particularly limited. The inventors demonstrate below the efficacy of the method on fumonisin B1 and fumonisin B2, both of which are produced by *Fusarium* fungal pathogens and are common contaminants of corn products. Fumonisin is just an exemplary toxin. The organic toxins synthesized by moulds are of similar composition and structure, so the methods of the invention are also useful for the destruction of many other types of mycotoxins, including but not limited to aflatoxins, in particular Ochratoxin A, Deoxynivalenol, Zearalenone etc.

The treatment according to the methods of the invention are also useful for inactivation of the mould itself, i.e., disinfection of agricultural products contaminated with microorganisms, in particular for cases where the microorganisms are not hidden from plasma, i.e., not in gaps deep inside the agricultural products.

In view of the present methods, devices for the industrial processing of agricultural products, with suitable parts to implement the present methods, are enabled. At their broadest, such devices may simply include a discharge or treatment chamber configured to generate a hydrogen plasma at the required discharge power density.

For example, in a second aspect of the present invention, there is provided a device for detoxifying agricultural products contaminated with toxins, the device comprising: (a) a vacuum-tight discharge chamber which is controllably connected to a source of reactive hydrogen species and (b) an inductively coupled radiofrequency generator or a microwave generator, wherein the inductively coupled radiofrequency generator, where present, is connected to a coil via a matching network to sustain plasma within the discharge in use, the coil mounting on the discharge chamber, or wherein the microwave generator, where present, houses the discharge chamber.

The source of reactive hydrogen species may be, for example, a tank or reservoir containing such reactive species. The reactive species is the gas mixture at equilibrium conditions. For example, it may be a flask containing hydrogen. The flask may contain water vapour, hydrogen sulfide, or ammonia, or a mixture of one of these with hydrogen, a mixture of two or more of these, or a mixture of two or more of these with hydrogen. It may be connected to the discharge chamber by a conduit, through which the species can flow. The flow through the conduit may be controlled, for example to turn on and off or to adjust the flow rate.

The coil may have a similar size to the discharge chamber; in that case, substantially the entire discharge chamber can act as a treatment zone for the agricultural products. Alternatively, the coil may be smaller than the discharge chamber, such that within the chamber there are pre-treatment, treatment and post-treatment zones, for example. The chamber may have a separate inlet and outlet, through which agricultural products can be fed into and out of the discharge chamber respectively. Having been fed through the inlet, the products may in order enter the pre-treatment, treatment and post-treatment zones before being fed through the outlet and leaving the chamber.

The device may include a reservoir for containing contaminated agricultural products (that is, agricultural products contaminated with toxins) which is attached to the inlet, and a reservoir for containing detoxified agricultural products which is attached to the outlet. There may be plural such reservoirs for either contaminated agricultural products or detoxified agricultural products, each of which is attachable to the inlet or outlet as appropriate. At the contaminated agricultural products side, the inlet, this can allow a feed into the discharge chamber from a first feedstock reservoir, while a second feedstock reservoir is (re)filled; then, the feed can be switched so that the second feedstock reservoir is attached to the inlet while the first feedstock reservoir is refilled. Similarly, at the detoxified agricultural products side, the outlet, this can allow a feed out of the discharge chamber into a first product reservoir while a second product reservoir is emptied; then, the feed can be switched so that the second product reservoir is filled while the first product reservoir is emptied.

It will be apparent that there may be more than two feedstock or product reservoirs, and that the number of those is not dependent on how many of the others there are. The reservoirs may be of different sizes; or may be for containing different types of agricultural products.

The flow of agricultural products from the inlet to the outlet, through the treatment zone, may be achieved in various ways. A simple conveyor is possible. On the other hand, preferred may be a gravity driven drop through the treatment zone. In such a case the inlet is at a position above the outlet. There may be conveyors to catch the agricultural products that fall from the inlet down through the discharge chamber; once caught on the conveyor they are conveyed to the outlet.

A schematic of a preferred embodiment of the device is shown in Figure 1. Here there are two feedstock reservoirs, containers of contaminated products 1 and 2, which are filled with contaminated products. The containers of contaminated products 1 and 2 are evacuated, and the products are slowly transferred from the container of contaminated products 1 to the discharge chamber 3 using the transport belt 4. The inlet to which they are conveyed is at the top of the discharge chamber 3. Gaseous plasma is sustained within the discharge chamber 3 inside the coil 5 connected to the RF generator 6 via a matching network. The products are dropped from the top of the discharge chamber 3, so they fall through the plasma to the bottom side of the discharge chamber 3. While falling through the discharge chamber 3, toxins on the products are degraded due to the interaction with gaseous plasma. A transport belt 7 transports the decontaminated products through an outlet to a container of detoxified products 8. The process takes place until the container of the contaminated products 1 is empty, and the container of detoxified products 8 is full. Then, without disturbing the processing and without breaking vacuum conditions, agricultural products can then be transported from the container of the contaminated products 2 to the discharge chamber 3 using the transport belt 4. The products are again dropped from the top side of the discharge chamber 3, so they fall through the plasma to the bottom side of the discharge chamber 3. The toxins on the products are again degraded during falling through the discharge chamber 3 due to the interaction with the gaseous plasma. The transport belt 7 transports the decontaminated products through the outlet to the container of detoxified products 9. During the passing of products from the container of the contaminated products 2 through the plasma to the container of detoxified products 9, the container of the contaminated products 1 is filled with new contaminated products, and detoxified products are discarded from the container of detoxified products 8. These cycles can be repeated until all desired contaminated products are treated.

An appropriate configuration allows for sustaining uniform gaseous plasma in a large volume inside the coil in an almost pure inductive mode, i.e., the plasma is not in contact with any metallic part of the discharge chamber. The coil serves as an antenna and may be mounted onto a discharge chamber. The discharge chamber may be made from a dielectric material. The discharge tube may also be cylindrical in shape, such that individual products are treated uniformly when passing through the plasma column. A particularly useful configuration was found when the coil was mounted onto a cylindrical discharge tube made from quartz glass. Under the preferred conditions, the density of charged particles in such a plasma is close to or about 10¹⁹ m⁻³, thus assuring for extensive excitation of gaseous species by free electrons. The dissociation of gaseous molecules at such conditions is almost complete, thus assuring for a large flux of chemically reactive radicals onto the surface of the agricultural products. The synergy between the extensive radiation and the high flux of radicals causes extensive degradation of the toxins that are present on the surface of the agricultural products. The interior of the products is not affected much by the short treatment time in the plasma.

As explained above, preferably, the agricultural products may enter the discharge chamber from an inlet, pass through the plasma column and leave via an outlet, wherein the coil is positioned between the inlet and the outlet, such that, in use, a product can fall from the discharge chamber from the inlet to the outlet passing the location of the coil. In this instance the products are conveyed though the plasma by gravity; they might instead be mechanically conveyed for example using a transport belt.

It will be appreciated that, for the products to be dropped, the inlet may be at the first location and the outlet may be at the second position. A preferred position of the inlet, the outlet and the coil can be seen in Figure 1. The inlet and outlet may be separated by a distance greater than the length of the coil powered by the radiofrequency generator or microwave generator. As the agricultural products may be dropped through the plasma, the residence time of any product dropped upon vacuum conditions through gaseous plasma depends only on the length of the plasma column generated within the discharge chamber. The residence time may be equal to the treatment time. The plasma length may be almost equal to the length of the coil powered by the radiofrequency generator or microwave generator.

Preferably, the agricultural products are dropped through a cylindrical discharge tube, such that individual products are treated rather uniformly within the plasma column. Such a treatment assures for the destruction of a high-enough amount of toxins providing the concentration of toxins on the surface of agricultural products is moderate. In cases of highly contaminated agricultural products, a single passage of agricultural product through plasma may not assure for the desired level of detoxification, in particular for cases where toxins are unevenly distributed on the product surfaces and/or the initial concentration of the toxins is very high. In such cases, one could repeat the plasma treatments until the desired detoxification is achieved.

Accordingly the devices of the present invention may include a conduit for transporting products from the outlet back to the inlet. Alternatively, a container or reservoir of treated (detoxified) agricultural products can simply be moved and used as a feedstock reservoir.

The skilled person may employ the present method of detoxifying agricultural products using the present device for detoxifying agricultural products. The preferred embodiments of the method may be employed for the operation of the device. That is, the various parts of the device may be adapted or configured to provide the plasma conditions and other features of the methods set out herein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

Example 1 and Comparative Example A herein demonstrate the effect of the identity of the gas on the concentration of toxins in corn.

### EXAMPLE 1

Example 1 demonstrates the effect of hydrogen as the sole gas on the concentration of toxins in corn.

Treatment was carried out using a device as schematically illustrated in Figure 1. The entire system in Figure 1 is hermetically tight. There are four containers, two for contaminated products and two for plasma-treated products. The containers of the contaminated products 1 and 2 were filled with corn. The corn was intentionally contaminated with fumonisin B1 and fumonisin B2 toxins at the concentration of 2000 and 350 mg/kg, respectively. The contaminated corn was gradually transferred from container of contaminated products 1 to the container of detoxified products 8 through the discharge chamber 3 using the transport belts 4 and 7 at the rate of about 1000 kg/h. Gaseous plasma was sustained within the discharge chamber 3 inside the coil 5 connected to the radiofrequency generator 6 via a matching network (not shown in Figure 1). The matching network enabled automatic matching of the radiofrequency generator. The generator operated in the continuous mode at the frequency of 150 kHz and output power of 40 kW. The volume of the discharge chamber 3 was about 50 litres, but the luminous plasma expanded in a volume of about 20 litres. The power density was thus about 2000 W per litre. The products were moved to the upper side of the discharge chamber 3 by the transport belt 4, so they fell through the plasma to the bottom side of the discharge tube. The residence time of the corn falling through plasma inside the discharge chamber 3 was about 0.3 s. Hydrogen was used as a reactive gas, and the pressure was about 80 Pa. While falling through the discharge chamber 3, the toxins on the products were partially degraded because of the interaction with gaseous plasma. The concentration of both toxins (fumonisin B1 and B2) dropped below the detection limit, which was about 100 µg/kg. The temperature of the corn after it was collected in the container of detoxified products 8 increased less than 5 K. After plasma treatment, the concentration of toxins on the seeds was determined using Liquid Chromatography with tandem mass spectrometry (LC-MS/MS). Briefly, after plasma treatment, seeds were ground to a particle size of 1 mm using a laboratory mill Retsch ZM 100 (Haan, Germany). Ten grams of a sample were shaken with 100 mL of an acetonitriledeionised water mixture (84 + 16) for 1 h using an IKA HS 501 digital linear shaker (IKA Labortechnik, Staufen, Germany). A total of 4 mL of the filtered extract was evaporated under vacuum to dryness using a Syncore Polyvap system (Büchi, Flawil, Switzerland). For mycotoxin concentrations above the calibration range, the filtered extracts were diluted for further work. The dry residue was reconstituted in 0.5 mL of a methanol-deionised water mixture (20 + 80). An aliquot - 10 µL of the solution - was injected into the UPLC- MS/MS system (Acquity UPLC H Class system) coupled with a triple-quadrupole mass spectrometer (Xevo TQ MS) equipped with an electrospray ionization (ESI) interface and MassLynx software for data collection and processing (Waters, Milford, MA, USA). The vials were kept in the autosampler at 15 °C. For the matrix-matched calibration, 4 mL portions of the filtered extracts were spiked with the appropriate amounts of standard solutions and prepared along the samples.

Mixed trichothecene standard solution in acetonitrile (AFG1, AFG2, AFB1 and AFB2) produced by Trilogy (Washington, MO, USA) were used. Mixed working standard solutions were prepared in acetonitrile and stored in amber glass vials at -20 °C. The concentrations of stock standard solutions were 0.5 µg/mL. Acetonitrile, methanol, acetic acid (Sigma-Aldrich, Steinheim, Germany), and ammonium acetate (Merck, Darmstadt, Germany) were p.a. or LC-MS grade purity. Deionized water was prepared using a Milli-Q system (Millipore, Bedford, MA, USA).

### EXAMPLE 2

The experiment as disclosed in Example 1 was repeated with the same device (as shown in Figure 1), except that water vapour was used instead of hydrogen as the working gas. In this case, the concentration of fumonisin B2 decreased below the detection limit, while the concentration of fumonisin B1 decreased to 250 µg/kg. The somewhat lower degradation rate as compared to plasma sustained in hydrogen is explained by less intensive radiation of water-vapour plasma (which still equates to a plasma sustained in an atmosphere comprising hydrogen; just less hydrogen) as compared to plasma sustained in almost pure hydrogen. Hence the use of higher proportions of hydrogen, and even pure hydrogen, is preferred in the present invention.

## Claims

1. A method for detoxifying agricultural products contaminated with toxins, the method comprising subjecting the products to gaseous plasma sustained in an atmosphere comprising hydrogen, the plasma being sustained at a discharge power density of 500 to 10000 W/litre at a gas pressure of 1 to 10000 Pa.

2. A method according to claim 1, wherein the plasma is sustained at a discharge power density of 1000 to 4000 W/litre.

3. A method according to claim 1 or claim 2, wherein the plasma is sustained at a gas pressure of 10 to 1000 Pa, more preferably 30 to 100 Pa.

4. A method according to any preceding claim, wherein the plasma is sustained with an inductively coupled radiofrequency discharge, such that plasma discharge occurs in the H-mode, or with a microwave discharge.

5. A method according to any preceding claim, wherein the atmosphere for sustaining the plasma comprises at least 10 % hydrogen, preferably at least 50 % hydrogen, more preferably at least 80 % hydrogen, and most preferably is substantially pure hydrogen.

6. A method according to claim 5, wherein the atmosphere is derived from a hydrogen-containing gas selected from hydrogen, water vapour, hydrogen sulphide, and ammonia, or a mixture of two or more of these.

7. A method according to any preceding claim, wherein the time for which the products are subjected to the plasma is 0.1 to 100 s, preferably 0.3 to 3 s.

8. A method according to any preceding claim, wherein the method comprises dropping the products from a first position to a second position through a treatment zone, wherein while the products are falling through the treatment zone they are subjected to the plasma.

9. A method according to any preceding claim, wherein the agricultural products are seeds, kernel, grains, nuts and beans in any form, original, ground, or milled.

10. A device for detoxifying agricultural products contaminated with toxins, the device comprising:
(a) a vacuum-tight discharge chamber which is controllably connected to a source of reactive hydrogen species and
(b) an inductively coupled radiofrequency generator or a microwave generator,
wherein the inductively coupled radiofrequency generator, where present, is connected to a coil via a matching network to sustain plasma within the discharge in use, the coil mounting on the discharge chamber, or
wherein the microwave generator, where present, houses the discharge chamber.

11. A device according to claim 10, wherein the discharge chamber is made from a dielectric material.

12. A device according to claim 10 or 11, wherein the discharge chamber comprises an inlet and an outlet for passing through agricultural products, wherein the coil, where present, is positioned between the inlet and the outlet, such that, in use, a product can fall through the discharge chamber from the inlet to the outlet passing the location of the coil.

13. A device according to any of claims 10 to 12, wherein the discharge chamber has a cylindrical shape.
